# EUROPEAN PATENT SPECIFICATION

(11) **EP 0 611 123 B1**
(45) Date of publication and mention of the grant of the patent: **03.11.1999**
(21) Application number: 94300883.9
(22) Date of filing: 07.02.1994
(51) Int. Cl.: A61F 5/448, A61F 5/443

(54) **Flushable ostomy pouch**
Spülbarer Ostomiebeutel
Poche d'ostomie jetable

(30) Priority: 08.02.1993 US 14953
(43) Date of publication of application: 17.08.1994
(73) Proprietor: E.R. SQUIBB & SONS, INC., Princeton, New Jersey 08543-4000 (US)
(72) Inventor: Keyes, Denis E., Yardley, Pennsylvania 19067 (US); Johnsen, Kenneth A., Piscataway, New Jersey 08854 (US)
(74) Representative: Holmes, Miles Keeton

(56) References cited:
- EP-A- 0 227 317
- EP-A- 0 276 898
- DE-U- 9 217 414
- FR-A- 2 385 598
- GB-A- 1 256 866
- US-A- 4 490 145
- US-A- 4 681 574
- US-A- 4 917 690

## Description

This invention is directed to ostomy pouches and more particularly to a novel flushable ostomy pouch.

One of the problems associated with ostomy care is the disposal of the ostomy collection pouch after it has been used. If the used pouch is disposed of by flushing down a toilet, there is a risk that the pouch may become trapped in a toilet passage or sewer line, thereby causing plumbing problems. Thus some users empty the contents of the pouch into the toilet and then discard the pouch in the garbage.

Other users dispose of the used pouch and its contents in the garbage, which usually necessitates prewrapping of the pouch with paper and/or placement of the used pouch in a plastic bag prior to disposal. Regardless of which measures are taken to dispose of a used ostomy pouch, the process is generally unduly laborious and oftentimes discomforting.

Thus there has been an ongoing effort to develop an ostomy pouch that provides relatively trouble-free flushability down a toilet.

A major problem in flushing an ostomy pouch down a toilet is that the coupling or securing structure around the waste inlet opening of the ostomy pouch, such as shown in U.S. Patent 4,372,308, can cause the pouch to become trapped in the flow passages of the toilet or in a connecting pipe or sewer line.

Efforts have thus been made to form ostomy pouches of materials that soften and become slimy or slippery when contacted with water to promote flowage in pipelines and flow passages.

While pouches that become slimy or slippery upon contact with water help minimize clogging and trapping problems associated with flush disposal of ostomy pouches, they can be discomforting if they become wet while being worn. Such pouches might discourage a user from engaging in swimming and other physical activity and would require protective covering while showering. Furthermore, such pouches may still cause clogging in toilets with relatively low volume flush capacity.

Another structure that facilitates flush disposal of ostomy pouches is that of U.S. Patent 4,830,187, which shows a carrier sleeve or bag into which a pouch can be placed before flush disposal. The sleeve or bag forms a slimy or slippery layer when exposed to water, thereby sliding on surfaces that might otherwise cause snagging of the pouch. However, since the carrier sleeve conforms to the pouch during flushing, a pouch with a coupling that is not flexible enough to negotiate the flow passages in toilet may still become trapped even with a slippery carrier sleeve.

Reference is also made to EP-A-0276898 which discloses an ostomy appliance including a bag for receiving discharged wastes. There is a stomal orifice in one wall of the bag and a washer attached to the wall of the bag and having a flat surface intended to face towards the body of the wearer when the appliance is worn, surrounding said orifice. A medical grade adhesive pad carries a flange and has an inner peripheral rib. Adhesive means is used for adhesively attaching said bag to said flange. The washer further comprises a flexible seal strip at the inner margin of said washer surrounding said stomal orifice.

Reference is also made to US-A-4490145 which describes an ostomy pouch having a filter element affixed to the outer pouch wall. The outer pouch wall has an aperture and the filter element includes a polymeric film cover and an insert of gas deodorising material. The film cover also has an aperture and opposite ends of the insert overlie the two apertures. In some embodiments, the pouch can be pear shaped, with the wider edge uppermost.

It remains desirable to provide an ostomy pouch that can be adapted to be easily flushed down a toilet, even a water-saver toilet, and which has an optimum height, width, and convergence angle to facilitate flush disposal.

Aspects of the present invention are defined in the claims.

In a preferred embodiment, a flushable ostomy pouch includes an envelope formed of flexible plastic sheet material that defines a waste collection chamber for body waste that passes through a stoma. A waste inlet opening is formed in the envelope for passage of waste material from the stoma into the collection chamber. Coupling means are provided on the envelope around the waste inlet opening for positioning the waste inlet opening around the stoma. The coupling means can be formed of molded annular plastic to mechanically interengage with a complementary shaped coupling portion provided around the stoma.

The coupling member is detachably bonded to the envelope so as to permit removal from the envelope prior to flushing the pouch in a toilet. The bonding agent which joins the coupling member to the envelope has a predetermined bond strength that permits the coupling member to be peeled as a unit from the envelope. Thus the removal of the coupling means from the pouch facilitates flush disposal of the envelope even in a water-saver toilet which has less water volume flush capacity than a standard toilet, and minimizes the likelihood that the flushing of the pouch will result in trappage within the sewer line.

The removed coupling member can be discarded or reused on a replacement pouch.

In European wash down water closets, when the pouch is ready for disposal, the top portion of the pouch can be cut or otherwise ripped to permit evacuation of the contents of the collection chamber during the flush process.

Preferably the envelope has opposite side edges that converge from the top portion of the envelope to the bottom portion such that the envelope in plan view is substantially V-shaped. The top width, pouch height and convergence angle are of a predetermined magnitude to assure optimum flushibilty of the pouch. The pouch is placed bottom end down and can be deposited in a toilet with a sleeve or bag that becomes slippery upon contact with water.

Among the several advantages achievable by the invention may be noted the provision of a novel ostomy pouch, a novel ostomy pouch that can be disposed of by flushing down a water-saver toilet, a novel ostomy pouch with a detachable coupling member, a novel ostomy pouch with a detachable coupling member that can be separated from the pouch prior to disposal of the pouch, a novel ostomy pouch having a coupling member that can be separated from the pouch as a unit without damaging the pouch to facilitate flush disposal of the pouch, a novel ostomy pouch with a detachable coupling member that can be reused, a novel ostomy pouch which has a converging streamlined shape from top to bottom with an optimum angle of convergence, optimum width, and optimum height to facilitate flush disposal in a water-saver toilet, and a novel method for facilitating flush disposal of an ostomy pouch.

An embodiment of the invention is now described by way of example only, with reference to the accompanying drawings, in which:
FIG. 1 is a simplified perspective view of an ostomy pouch incorporating one embodiment of the invention, prior to being coupled to a support flange provided around the stoma;
FIG. 2 is a simplified perspective view thereof after the pouch has been coupled to the support flange;
FIG. 3 is a plan view thereof;
FIG. 4 is a sectional view thereof, taken on the line 4-4 of FIG. 3;
FIG. 5 is a sectional view thereof, taken on the line 5-5 of FIG. 2;
FIG. 6 is a view similar to FIG. 5, showing the pouch, after use, uncoupled from the support flange; and
FIG. 7 is a simplified perspective view thereof, after the coupling member and the pouch envelope have been separated.

Corresponding reference characters indicate corresponding parts throughout the several views of the drawings.

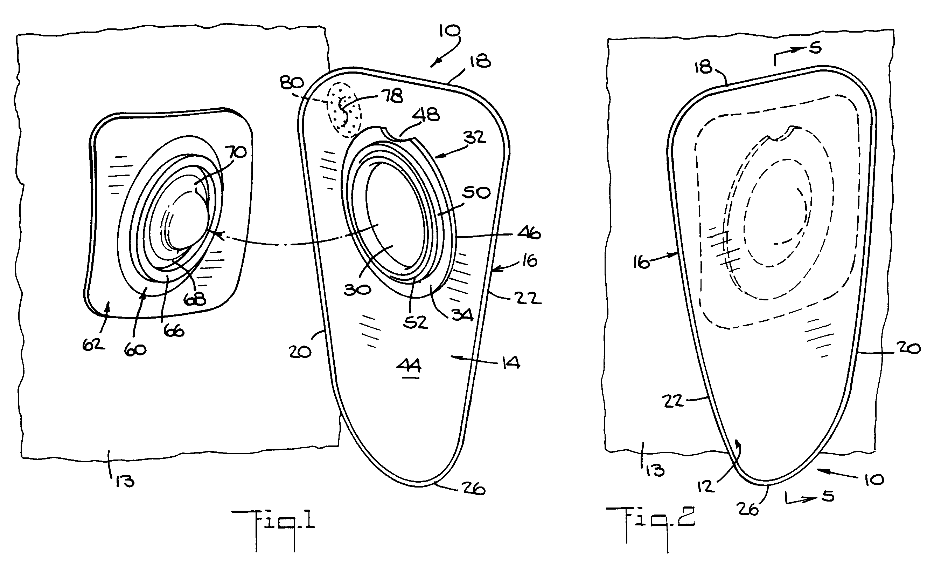

An ostomy pouch incorporating one embodiment of the invention is generally indicated by the reference number 10 in Fig. 1. The pouch 10 is formed of a suitable known thermoplastic material that is gas and water impermeable, flexible and expandable.

The pouch 10 includes a front wall 12 that faces away from the abdomen 13, and a rear wall 14 that confronts the abdomen 13, joined together by a peripheral thermoweld 16. The pouch 10 further includes a top portion 18 with rounded corners, and opposite side portions 20 and 22 that converge from the top portion 18 to a rounded bottom portion 26.

In a preferred embodiment of the pouch 10, the height of the pouch is approximately 197mm (7.750 inches), the maximum width of the top portion 18 at the rounded corners is approximately 130mm (5.125 inches), the bottom portion 26 has a radius of approximately 44mm (1.750 inches) and the angle of convergence of the side portions 20 and 22 is approximately 15°. The walls 12 and 14 are approximately 0.43 to 1.14mm (0.017 to 0.045 inches) thick.

A preferred size range for the pouch 10 is approximately 114 to 152mm (4.5 to 6 inches) maximum width of the pouch, approximately 152 to 203mm (6 to 8 inches) pouch height and a convergence angle of approximately 15° to 25°.

A waste inlet opening 30 is formed in the rear wall 14 nearer the top portion 18 of the pouch 10 than the bottom portion 26. The waste inlet opening 30 is bordered by a plastic coupling ring 32 (Fig. 7) having a base portion 34 and a bottom surface 36.

A contact adhesive 38 (Fig. 7) is provided on an outside surface 44 of the rear wall 14 in the form of a washer-shaped wafer approximately 0.25 to 0. 38mm (0.010 to 0.015 inches) thick, to bond the bottom surface 36 of the coupling ring 32 to the rear wall 14. Preferably the adhesive 30 is of a type that securely joins the coupling ring 32 to the rear wall 14 to prevent axial removal of the ring 32. However, the adhesive is selected to permit peeling of the coupling ring 32 from the rear wall 14 without damaging the rear wall.

A suitable adhesive 38 is a hydrocolloid adhesive such as Stomahesive®, manufactured by Bristol-Myers Squibb Company. The adhesive wafer can be covered with a known silicone release paper (not shown) that is removed prior to installation of the coupling ring 32. Thus the coupling ring 32 can be easily positioned on the rear wall 14 by the user or the manufacturer.

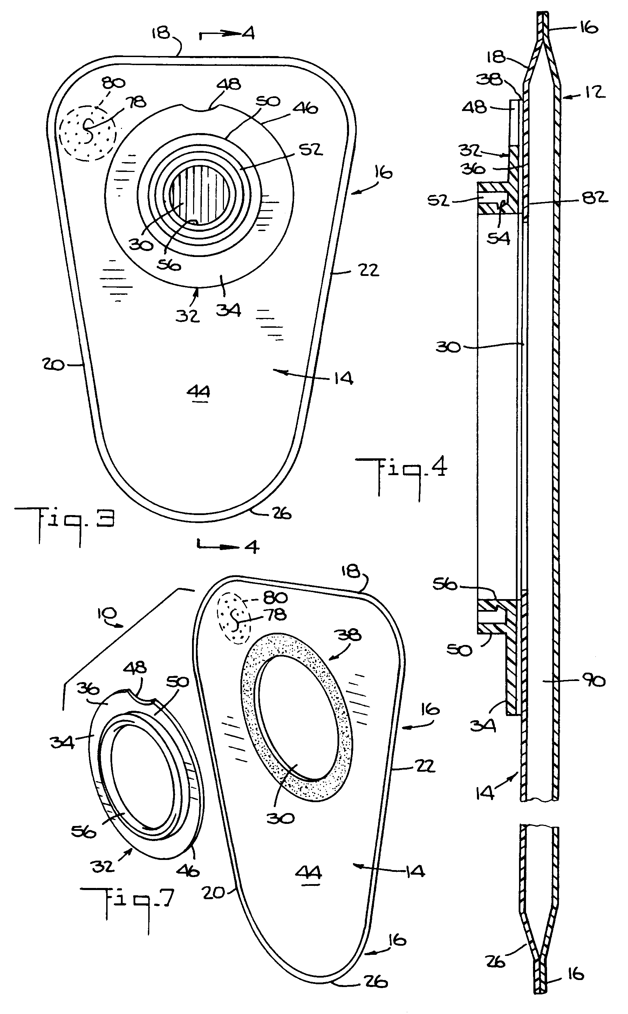

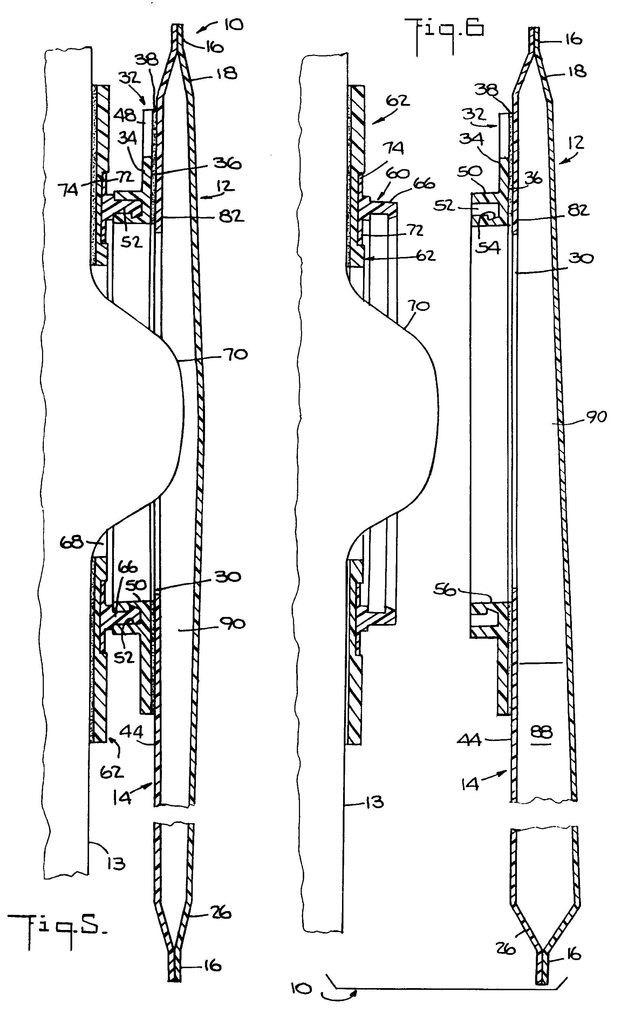

The base portion 34 of the coupling ring 32 has a peripheral edge 46 with a generally concave touch indicator notch or recess 48. Preferably the coupling ring 32 is positioned such that the recess 48 is at the 12 o'clock position as shown in Fig. 4. The indicator notch 48 permits touch detection of an initial peel section of the coupling ring 32 at the area of the notch 48.

The coupling ring 32 further includes an annular rim 50 projecting away from the base portion 34. An annular engagement slot 52 formed in the rim 50 includes an undercut latch portion 54. An inner peripheral surface 56 of the rim 50 encircles the waste inlet opening 30 at the rear wall 14.

Referring to Figs. 1, 5 and 6, the coupling ring 32 is adapted to interlock with a complementary shaped coupling ring 60 on a mounting plate 62 which is adapted to adhere to the abdominal wall 13 in any suitable known manner. A central opening 68 in the mounting plate 62, which aligns with the stoma 70. is surrounded by an annular latch projection 66 that interlocks in the engagement slot 52 of the coupling ring 32.

Preferably the coupling ring 60 and the mounting plate 62 are formed as a two-part structure wherein an annular base portion 72 of the coupling ring 60 is bonded to an annular channel 74 of the mounting plate 62.

An S-shaped gas evacuation slit 78 or other suitable gas evacuation outlet is formed in the rear wall portion 14 of the pouch 10 near the top and side edges 18 and 20, offset from the coupling ring 32. A generally circular deodorizing filter 80 of the type shown in U.S. Patent 5,074,851, is provided at an inside surface 82 of the rear wall 14 in substantial alignment with the gas evacuation slit 78.

In using the ostomy pouch 10, the mounting plate 62 is first adhered to the abdominal wall 13 to align the central opening 68 with the stoma 70. The coupling ring 32 is installed on the rear wall 14 of the pouch 10 and engaged with the coupling ring 60 of the mounting plate 62 to interlock the latch projection 66 in the engagement slot 52 as shown in Fig. 5. A leak tight joint is thus established around the stoma 70 which also aligns with the waste inlet opening 30 of the ostomy pouch 10.

Waste material 88 (Fig. 6) that issues from the stoma 70 passes into a waste collection chamber 90 of the pouch 10. When an adequate amount of the waste material 88 accumulates in the collection chamber 90, the pouch 10 is ready for disposal.

To facilitate flush disposal of the used pouch, the welded wall portions 12 and 14 which constitute the envelope 12-14 of the pouch are separated from the coupling ring 32. Separation of the coupling ring 32 can be accomplished by peeling the rear wall 14 away from the base portion 36 of the coupling ring 32 while the coupling ring 32 is engaged with the coupling ring 60.

The adhesive bond between the couplinq ring 32 and the tape 38 is of a predetermined strength that can be overcome by peeling without causing separation of the coupling rings 32 and 60. The peeling operation is initiated at the touch indicator notch 48 which is sized to permit finger engagement with the coupling ring 32.

Separation of the envelope 12-14 from the coupling ring 32 can also be accomplished by initially disengaging the coupling rings 32 and 60 and then peeling the ring 32 from the rear wall 14. Coupling disengagement is obtained by pressing a finger between the couplings 32 and 60 to release the pouch 10 from the mounting plate 62. The coupling ring 32 can then be peeled from the rear wall 14 while the ostomy pouch 10 is held at the top portion 18.

Once the coupling ring 32 is separated from the rear wall 14, the envelope 12-14, minus the coupling ring 32, can be flushed down a water-saver toilet, preferably with a carrier sleeve of the type shown in U.S. Patent 4,830,187. In European wash down water closets, the carrier sleeve can be omitted. However, the top portion 18 is preferably cut or ripped to permit evacuation of confined waste during the flushing process.

The envelope 12-14 is deposited in a toilet, bottom portion first. The convergent shape of the pouch 10 and the non-obtrusive adhesive wafer 38 enable the envelope 12-14 to flow in streamlined fashion through the toilet passages and sewer pipes. It has been found that flushability of the pouch 10 is enhanced when the pouch has a 15° to 25° angle of convergence and the width and pouch height are in the size ranges previously specified.

The removed coupling ring can be separately discarded in a garbage container or reused on a replacement envelope 12-14 that has an adhesive wafer 38 covered by silicone release paper. The release paper is thus removed and the coupling 32 installed. The replacement pouch 10 can then be interlocked with the coupling 60 on the abdominal wall 13.

Some advantages of the present invention evident from the foregoing description include an ostomy pouch that has a molded plastic coupling ring that can be removed from the pouch envelope to facilitate flush disposal of the envelope in a water-saver toilet. Removal of the coupling ring is easily accomplished by peeling from the pouch wall. The removed coupling ring can either be reused or discarded. The pouch envelope can be supplied to the user without the coupling ring so as to permit reuse of a single coupling ring. The V-shaped profile of the pouch, the thin walled structure of the pouch envelope, and the thin gauge of the adhesive wafer on the pouch envelope and the predetermined size range of pouch height, top width and convergence angle ensure that the pouch structure itself will not constitute an obstacle to flushability of the pouch, even in low flush volume toilets. The pouch thus provides substantially risk-free flush disposal capability and eliminates the need to resort to garbage disposal of a used ostomy pouch.

In view of the above, it will be seen that advantageous results are attained.

As various changes can be made in the above constructions and method without departing from the scope of the invention, it is intended that all matter contained in the above description or shown in the accompanying drawings shall be interpreted as illustrative and not in a limiting sense.

## Claims

1. A flushable ostomy pouch (10) for holding body waste that passes through a stoma comprising,
a) a envelope (12,14) formed of flexible plastic sheet material defining a waste collection chamber for body waste that passes through a stoma,
b) a waste inlet opening (30) formed in said envelope for passage of waste material from said stoma into said collection chamber,
c) flexible pouch-side coupling means (32) on said envelope around said waste inlet opening for co-operating with a body-side coupling means for attaching said waste inlet opening around a stoma,
d) bonding means (38) for detachably bonding said coupling means (32) to said envelope, characterised in that said pouch-side coupling means (32) is removable from said envelope to facilitate flush disposal of said envelope in a toilet without coupling means (32) attached thereto, said bonding means (38) having a predetermined bond strength that permits said coupling means (32) to be peeled as a unit from said envelope.

2. The ostomy pouch as claimed in claim 1 wherein said envelope has a top end portion (18), a bottom end portion (26) opposite said top end portion, and opposite side edges (20,22) extending between said top end portion (18) and said bottom end portion (26), said side edge portions (20,22) converging toward each other in a direction from said top end portion to said bottom end portion such that said envelope in plan view is substantially V-shaped.

3. The ostomy pouch as claimed in claim 1 wherein said coupling means (32) includes a coupling member (32) ad means (50-56) for securing said coupling member around a stoma in a fixed position.

4. The ostomy pouch as claimed in claim 3 wherein said coupling member is s molded annular plastic member (32) formed with a coupling portion (50-56), whereby said coupling portion is interengagable with a complementary-shaped coupling portion provided around the stoma.

5. The ostomy pouch as claimed in claim 4 wherein said annular plastic member (32) has a peripheral edge portion (48) formed to permit bending of said edge portion away from said envelope to initiate peeling of said annular plastic member from said envelope.

6. The ostomy pouch as claimed in claim 5 wherein said annular plastic member includes indicator means (48) at said peripheral edge to indicate where the annular plastic member is to be initially peeled from said envelope.

7. The ostomy pouch as claimed in claim 6 wherein said indicator means is formed with a touch detector (48) to permit touch detection of said initial peel section.

8. The ostomy pouch as claimed in claim 7 wherein said touch detector is a recess (48) in said peripheral edge.

9. An ostomy pouch according to any preceding claim, wherein said envelope has a top end portion (18), a bottom end portion (26) opposite said top end portion, and opposite side edges (20, 22) extending between said top end portion (18) and said bottom end portion (26), said side edge portions (20, 22) converging toward each other in a direction from said top end portion to said bottom end portion such that said envelope in plan view is substantially V-shaped with an angle of convergence in the range of approximately 15° to 25°

10. A method for facilitating flush disposal of an ostomy pouch comprising,
(a) forming an envelope of flexible plastic sheet material (12, 14) with a waste inlet opening (30) and a waste collection chamber,
(b) bonding a flexible pouch-side coupling member (32) onto the envelope around the waste inlet opening (30) for co-operating with a body-side coupling member to position the waste inlet opening around a stoma, characterised in that the pouch-side coupling member is detachably bonded to the envelope, and in that the method further comprises:
(c) removing the flexible coupling member (32) from the envelope when the envelope is ready for disposal to permit flushing of the envelope down a toilet without any coupling member attached thereto.

11. The method of claim 10, wherein the coupling member (32) is removed from the envelope by peeling the envelope away from the coupling member.

12. The method of claim 10, wherein the coupling member (32) is removed from the envelope by peeling the coupling member away from the envelope.

13. The method of claim 10 wherein the coupling member (32) is joined to the envelope by an adhesive tape (38) that is joined to the envelope at a non-adhesive side and which holds the coupling member it the adhesive side.

14. The method of claim 10 wherein the removal of the coupling member from the envelope is initiated at an initial peel section that is indicated with a touch detector (48).

15. The method of claim 10 including depositing the envelope (12,14) in a V-shape.

16. The method of claim including depositing the envelope in a carrier sleeve that becomes slippery upon immersion in water.

17. The method of claim 10 including severing the top portions (18) of the envelope.

18. The method of claim 10, further comprising the step of disposing of the pouch by inserting the envelope into a toilet after removal of the flexible coupling member (32), and flushing the toilet.

## Patentansprüche

1. Wegspülbarer Ostomiebeutel (10) zum Halten von durch ein Stoma fließendem Körperausscheidungsmaterial mit:
a) einer aus einem flexiblen lagenförmigen Kunststoffmaterial gebildeten Hülle (12, 14), die eine Ausscheidungsmaterialsammelkammer für durch ein Stoma fließendes Körperausscheidungsmaterial definiert;
b) einer in der Hülle ausgebildeten Ausscheidungsmaterialeinlaßöffnung (30), durch die das Ausscheidungsmaterial vom Stoma in die Sammelkammer fließt;
c) einer auf der Hülle um die Ausscheidungsmaterialeinlaßöffnung angeordneten, flexiblen, beutelseitigen Kupplungseinrichtung (32) für eine Zusammenwirkung mit einer körperseitigen Kupplungseinrichtung zum Befestigen der Ausscheidungsmaterialeinlaßöffnung um das Stoma; und
d) einer Verbindungseinrichtung (38) zum lösbaren Verbinden der Kupplungseinrichtung (32) mit der Hülle;
dadurch gekennzeichnet, daß
die beutelseitige Kupplungseinrichtung (32) von der Hülle entfernbar ist, so daß die Hülle in einer Toilette weggespült werden kann, ohne daß die Kupplungseinrichtung (32) daran befestigt ist, wobei die Verbindungeinrichtung (38) eine vorgegebene Haftfestigkeit aufweist, durch die ermöglicht wird, daß die Kupplungseinrichtung (32) als Einheit von der Hülle abgezogen werden kann.

2. Ostomiebeutel nach Anspruch 1, wobei die Hülle einen oberen Endabschnitt (18), einen dem oberen Endabschnitt gegenüberliegenden unteren Endabschnitt (26) und gegenüberliegende Seitenrandabschnitte (20, 22) aufweist, die sich zwischen dem oberen Endabschnitt (18) und dem unteren Endabschnitt (26) erstrecken, wobei die Seitenrandabschnitte (20, 22) in Richtung vom oberen Endabschnitt zum unteren Endabschnitt zueinander hin konvergieren, so daß die Hülle in Draufsicht betrachtet im wesentlichen V-förmig ist.

3. Ostomiebeutel nach Anspruch 1, wobei die Kupplungseinrichtung (32) ein Kupplungselement (32) und eine Einrichtung (50-56) zum Sichern des Kupplungselements in einer fixierten Position um ein Stoma aufweist.

4. Ostomiebeutel nach Anspruch 3, wobei das Kupplungselement ein geformtes oder gegossenes ringförmiges Kunststoffelement (32) mit einem Kupplungsabschnitt (50-56) ist, wobei der Kupplungsabschnitt mit einem um das Stoma ausgebildeten, komplementär geformten Kupplungsabschnitt in Eingriff gebracht werden kann.

5. Ostomiebeutel nach Anspruch 4, wobei das ringförmige Kunststoffelement (32) einen am Umfang angeordneten Randabschnitt (48) aufweist, der dazu geeignet ist, von der Hülle weggebogen zu werden, um den Vorgang zum Abziehen des ringförmigen Kunststoffelements von der Hülle einzuleiten.

6. Ostomiebeutel nach Anspruch 5, wobei das ringförmige Kunststoffelement eine am Umfangsrand angeordnete Anzeigeeinrichtung (48) aufweist, um anzuzeigen, wo das ringförmige Kunststoffelement anfangs von der Hülle abgezogen werden soll.

7. Ostomiebeutel nach Anspruch 6, wobei die Anzeigeeinrichtung eine Berührungsanzeigeeinrichtung (48) aufweist, um den anfangs abzuziehenden Abschnitt durch Berührung zu erfassen.

8. Ostomiebeutel nach Anspruch 7, wobei die Berührungsanzeigeeinrichtung eine Vertiefung (48) im Umfangsrand ist.

9. Ostomiebeutel nach einem der vorangehenden Ansprüche, wobei die Hülle einen oberen Endabschnitt (18), einen dem oberen Endabschnitt gegenüberliegenden unteren Endabschnitt (26) und gegenüberliegende Seitenrandabschnitte (20, 22) aufweist, die sich zwischen dem oberen Endabschnitt (18) und dem unteren Endabschnitt (26) erstrecken, wobei die Seitenrandabschnitte (20, 22) in Richtung vom oberen Endabschnitt zum unteren Endabschnitt zueinander hin konvergieren, so daß die Hülle in Draufsicht betrachtet im wesentlichen V-förmig ist, wobei ein Konvergenzwinkel 15° bis 25° beträgt.

10. Verfahren zum Ermöglichen der Entsorgung eines Ostomiebeutels durch Wegspülen mit den Schritten:
(a) Ausbilden einer Ausscheidungsmaterialeinlaßöffnung (30) und einer Ausscheidungsmaterialsammelkammer in einer Hülle aus einem flexiblen Kunststoffmaterial (12, 14);
(b) Verbinden eines flexiblen beutelseitigen Kupplungselements (32) um die Ausscheidungsmaterialeinlaßöffnung (30) mit der Hülle für eine Zusammenwirkung mit einem körperseitigen Kupplungselement, um die Ausscheidungsmaterialeinlaßöffnung um ein Stoma anzuordnen,
dadurch gekennzeichnet, daß
das beutelseitige Kupplungselement lösbar mit der Hülle verbunden ist und das Verfahren ferner aufweist:
(c) Entfernen des flexiblen Kupplungselements (32) von der Hülle, wenn die Hülle entsorgt werden soll, so daß die Hülle in einer Toilette weggespült werden kann, ohne daß ein Kupplungselement daran befestigt ist.

11. Verfahren nach Anspruch 10, wobei das Kupplungselement (32) durch Abziehen der Hülle vom Kupplungselement weg von der Hülle entfernt wird.

12. Verfahren nach Anspruch 10, wobei das Kupplungselement (32) durch Abziehen des Kupplungselements von der Hülle weg von der Hülle entfernt wird.

13. Verfahren nach Anspruch 10, wobei das Kupplungselement (32) durch ein Klebeband (38) mit der Hülle verbunden ist, das an einer nicht haftenden Seite mit der Hülle verbunden ist und das Kupplungselement an der Klebeseite hält.

14. Verfahren nach Anspruch 10, wobei mit der Entfernung des Kupplungselements von der Hülle an einem anfangs abzuziehenden Abschnitt begonnen wird, der durch eine Berührungsanzeigeeinrichtung (48) angezeigt wird.

15. Verfahren nach Anspruch 10 mit dem Schritt zum Ausbilden der Hülle (12, 14) in einer V-Form.

16. Verfahren nach Anspruch 10 mit dem Schritt zum Anordnen der Hülle in einer Trägerumhüllung, die durch Eintauchen in Wasser gleitfähig wird.

17. Verfahren nach Anspruch 10 mit dem Schritt zum Abtrennen des oberen Abschnitts (18) der Hülle.

18. Verfahren nach Anspruch 10, ferner mit den Schritten zum Entsorgen des Beutels durch Einbringen der Hülle in eine Toilette, nachdem das flexible Kupplungselement (32) entfernt wurde, und Spülen der Toilette.

## Revendications

1. Poche de stomie (10) jetable dans les cuvettes de W.C., destinée à retenir les excréments qui passent par un anus artificiel, comprenant:
a) une enveloppe (12, 14) formée d'une feuille de matière plastique souple délimitant un compartiment de recueil des déchets pour les excréments qui passent par un anus artificiel,
b) une ouverture (30) d'admission des déchets, formée dans ladite enveloppe pour permettre le passage des excréments dudit anus artificiel dans ledit compartiment de recueil,
c) un raccord (32) côté poche, souple, sur ladite enveloppe autour de ladite ouverture d'admission des déchets, destiné à coopérer avec un raccord côté corps pour fixer ladite ouverture d'admission des déchets autour d'un anus artificiel,
d) un moyen de liaison (38) destiné à lier de manière amovible ledit raccord (32) à ladite enveloppe, caractérisée en ce que ledit raccord côté poche (32) peut être séparé de ladite enveloppe pour faciliter l'élimination de ladite enveloppe dans une cuvette de W.C. sans le raccord (32) qui lui est fixé, ledit moyen de liaison (38) ayant une force de liaison prédéterminée qui permet audit raccord (32) d'être décollé d'un seul coup de ladite enveloppe.

2. Poche de stomie selon la revendication 1, dans laquelle ladite enveloppe a une partie supérieure (18), une partie inférieure (26) à l'opposé de ladite partie supérieure, et des bords latéraux opposés (20, 22) entre ladite partie supérieure (18) et ladite partie inférieure (26), lesdits bords latéraux (20, 22) convergeant l'un vers l'autre dans la direction de ladite partie supérieure à ladite partie inférieure, de telle sorte que ladite enveloppe, vue en plan, est sensiblement en forme de V.

3. Poche de stomie selon la revendication 1, dans laquelle ledit raccord (32) comporte un élément de raccord (32) et des moyens (50-56) pour fixer ledit élément de raccord autour d'un anus artificiel dans une position fixe.

4. Poche de stomie selon la revendication 3, dans laquelle ledit élément de raccord est un élément annulaire (32) en matière plastique moulée, qui est formé avec une partie de raccordement (50-56), de telle sorte que ladite partie de raccordement peut venir en prise mutuelle avec une partie de raccordement de forme complémentaire qui est prévue autour de l'anus artificiel.

5. Poche de stomie selon la revendication 4, dans laquelle ledit élément annulaire (32) en matière plastique comporte un bord périphérique (48) formé de façon à permettre la flexion dudit bord à l'opposé de ladite enveloppe pour amorcer le décollement dudit élément annulaire en matière plastique et de ladite enveloppe.

6. Poche de stomie selon la revendication 5, dans laquelle ledit élément annulaire en matière plastique comporte un indicateur (48) sur ledit bord périphérique pour indiquer l'endroit où l'élément annulaire en matière plastique doit commencer à être décollé de ladite enveloppe.

7. Poche de stomie selon la revendication 6, dans laquelle ledit indicateur comporte un détecteur à contact (48) pour permettre la détection par contact de ladite section de décollement initial.

8. Poche de stomie selon la revendication 7, dans laquelle ledit détecteur à contact est un évidement (48) pratiqué dans ledit bord périphérique.

9. Poche de stomie selon l'une quelconque des revendications précédentes, dans laquelle ladite enveloppe a une partie supérieure (18), une partie inférieure (26) à l'opposé de ladite partie supérieure, et des bords latéraux opposés (20, 22) entre ladite partie supérieure (18) et ladite partie inférieure (26) , lesdits bords latéraux opposés (20, 22) convergeant l'un vers l'autre dans la direction de ladite partie supérieure à ladite partie inférieure, de telle sorte que ladite enveloppe, vue en plan, est sensiblement en forme de V avec un angle de convergence compris entre 15° et 25° environ.

10. Procédé facilitant l'élimination d'une poche de stomie dans les cuvettes de W.C., consistant:
a) à former une enveloppe de feuille de matière plastique souple (12, 14), avec une ouverture (30) d'admission des déchets et un compartiment de recueil des déchets,
b) à lier un élément de raccord côté poche (32), souple, sur l'enveloppe autour de l'ouverture (30) d'admission des déchets, destiné à coopérer avec un élément de raccord côté corps de façon à positionner l'ouverture d'admission des déchets autour d'un anus artificiel, caractérisé en ce que l'élément de raccord côté poche est lié de manière détachable à l'enveloppe, et en ce que le procédé consiste en outre:
c) à retirer l'élément de raccord souple (32) de l'enveloppe quand l'enveloppe est prête à être jetée, de façon à permettre l'élimination de l'enveloppe dans une cuvette de W.C. sans aucun élément de raccord fixé à celle-ci.

11. Procédé selon la revendication 10, dans lequel on retire l'élément de raccord (32) de l'enveloppe en décollant l'enveloppe de l'élément de raccord.

12. Procédé selon la revendication 10, dans lequel on retire l'élément de raccord (32) de l'enveloppe en décollant l'élément de raccord de l'enveloppe.

13. Procédé selon la revendication 10, dans lequel l'élément de raccord (32) est réuni à l'enveloppe par un ruban adhésif (38) qui est réuni à l'enveloppe par une face non adhésive et qui maintient l'élément de raccord par sa face adhésive.

14. Procédé selon la revendication 10, dans lequel l'enlèvement de l'élément de raccord de l'enveloppe est amorcé au niveau d'une section de décollement initial qui est indiquée par un détecteur à contact (48).

15. Procédé selon la revendication 10, comprenant la réalisation de l'enveloppe (12, 14) en forme de V.

16. Procédé selon la revendication 10, comprenant le dépôt de l'enveloppe dans un manchon porteur qui devient glissant dès qu'il est plongé dans l'eau.

17. Procédé selon la revendication 10, comportant le sectionnement de la partie supérieure (18) de l'enveloppe.

18. Procédé selon la revendication 10, comprenant en outre l'opération qui consiste à se débarrasser de la poche en introduisant l'enveloppe dans une cuvette de W.C. après avoir enlevé l'élément de raccord souple (32), et à tirer la chasse.
